# EUROPEAN PATENT APPLICATION

(11) **EP 3 479 811 A1**
(43) Date of publication of application: **08.05.2019**
(21) Application number: 16907360.8
(22) Date of filing: 01.07.2016
(51) Int. Cl.: A61H 99/00

(54) **SWALLOWING TRAINING INSTRUMENT**

(71) Applicant: Hyogo College Of Medicine, Hyogo 663-8501 (JP); J Craft Co., Ltd., Osaka 594-1144 (JP); Eusense Medical Co., Ltd., Nishinomiya-shi, Hyogo 662-0052 (JP)
(72) Inventor: OKU, Yoshitaka, Nishinomiya-shi Hyogo 663-8501 (JP); UENO, Hiroshi, Izumi-shi Osaka 594-1144 (JP)
(74) Representative: Glawe, Delfs, Moll
(86) International application number: PCT/JP2016/069723
(87) International publication number: WO 2018/003127

(57) **Abstract**

A swallowing training apparatus 100 includes: a respiration detection part 116a configured to detect a respiratory motion of a subject; a swallowing induction part 116c configured to provide a trigger for swallowing induction to the subject on the basis of a detection result from the respiration detection part 116a; a swallowing detection part 116d configured to detect that swallowing was performed by the subject; and a determination output part 116e configured to determine whether or not the swallowing detected by the swallowing detection part 116d was at an appropriate timing with respect to the respiratory motion detected by the respiration detection part 116a, and configured to output information indicating a determination result, to outside.

## Description

### TECHNICAL FIELD

The present invention relates to a swallowing training apparatus for performing swallowing training.

### BACKGROUND ART

Many of pneumonias that cause deaths are aspiration pneumonias caused by so-called "aspiration". In particular, aspiration pneumonias in elderly persons are serious problems. "Aspiration" is a pathological condition in which swallowing cannot be appropriately performed and the thing having been taken in enters not the esophagus but the trachea. Especially as for elderly persons, prevention of aspiration pneumonia by inhibiting aspiration is an important issue.

In order to inhibit aspiration, it is effective to conduct training so as to become able to appropriately swallow. PATENT LITERATURE 1 below discloses a system for conducting swallowing training. In this system, a swallowing sign to a subject is started at the middle or near the middle of expiration, and a response of the subject to this sign is stored in this system.

### CITATION LIST

### [PATENT LITERATURE]

[PTL 1] Japanese National Phase PCT Laid-Open Publication No. 2013-508063

### SUMMARY OF THE INVENTION

### PROBLEMS TO BE SOLVED BY THE INVENTION

Generally, in many cases, aspiration occurs because the timings of swallowing and respiration are not appropriate. Therefore, in swallowing training, it is effective to conduct training such that swallowing is performed at an ideal timing during respiration.

However, usually, respiration is performed unconsciously, and thus, it is difficult for a subject to continue to be aware of his or her own respiratory motion during the training. In the case of PATENT LITERATURE 1 mentioned above, since a swallowing sign is given to the subject, the subject can understand the swallowing timing. However, it is difficult for the subject to confirm whether or not the swallowing performed in response to the sign was at an ideal timing. In particular, in the case of an elderly person, it is very difficult for the elderly person to determine, while being aware of his/her own respiratory motion, at what timing during respiration the elderly person performed swallowing. Therefore, only by giving a swallowing sign, swallowing training cannot be conducted effectively.

As for swallowing training, it is preferable that the subject conducts the training while confirming whenever necessary whether or not the subject performed swallowing at an ideal timing during respiration. It is also preferable that persons, such as speech-language-hearing therapists who instruct or supervise the swallowing training, can confirm whenever necessary whether or not the subject performed swallowing at an ideal timing during respiration.

The present invention has been made in consideration of such circumstances. An object of the present invention is to provide a swallowing training apparatus that enables a subject to effectively conduct swallowing training.

### SOLUTION TO THE PROBLEMS

A main mode of the present invention relates to a swallowing training apparatus for swallowing training. A swallowing training apparatus according to the present mode includes: a respiration detection part configured to detect a respiratory motion of a subject; a swallowing induction part configured to provide a trigger for swallowing induction to the subject on the basis of a detection result from the respiration detection part; a swallowing detection part configured to detect that swallowing has been performed by the subject; a swallowing determination part configured to determine whether or not the swallowing detected by the swallowing detection part has been at an appropriate timing with respect to the respiratory motion detected by the respiration detection part; and a result output part configured to output, to outside, information indicating a determination result, in accordance with determination having been performed by the swallowing determination part.

According to the swallowing training apparatus of the present mode, every time swallowing is performed by the subject in response to the trigger for swallowing induction, information indicating whether or not the swallowing was ideal is outputted to outside. Therefore, the subject can confirm, whenever necessary during the swallowing training, whether or not the swallowing performed by the subject was at an ideal timing. In addition, the speech-language-hearing therapist or the like can also confirm whenever necessary whether the subject performed swallowing at an ideal timing in the respiratory motion. Therefore, the subject can conduct the swallowing training smoothly and effectively by himself/herself or while being instructed by the speech-language-hearing therapist or the like.

The swallowing training apparatus according to the present mode may be configured to further include a respiration output part configured to output, to outside, information indicating the respiratory motion of the subject on the basis of the detection result from the respiration detection part. According to this configuration, the subject can be more clearly conscious of the respiratory motion, which is difficult for the subject to be conscious of, on the basis of the information outputted from the respiration output part. Thus, during the training, the subject can perform swallowing so as to be at an ideal timing with respect to the respiratory motion, while being clearly conscious of his/her own respiratory motion.

In this case, the swallowing training apparatus may be configured to further include a display configured to display information, wherein the respiration output part causes the display to display, as the information indicating the respiratory motion: first motion information indicating which of expiration and inspiration the respiratory motion is in; and second motion information indicating a progress state of expiration and inspiration. Accordingly, with reference to the display, the subject can understand which of expiration and inspiration his/her own respiration is in, and can understand the progress state of expiration and inspiration at each time point. Therefore, the subject can smoothly perform swallowing at an ideal timing in the respiratory motion, in response to the trigger from the swallowing induction part.

In this case, the respiration output part may be configured to cause display of at least one of the first motion information and the second motion information to be different between a case of the expiration and a case of the inspiration. Accordingly, the subject can more clearly understand which of expiration and inspiration the respiratory motion at the current time point is in, and can more smoothly perform swallowing in response to the trigger.

For example, the respiration output part may be configured to cause a color of the display of at least one of the first motion information and the second motion information to be different between the case of the expiration and the case of the inspiration. Accordingly, the subject can intuitively understand, by the color, which of expiration and inspiration the respiratory motion at the current time point is in.

In this case, preferably, the respiration output part sets the color of the display in the case of the expiration to a color (for example, green) indicating that the time is appropriate for swallowing, and sets the color of the display in the case of the inspiration to a color (for example, red) indicating that the timing is not appropriate for swallowing. Accordingly, the subject can intuitively understand, by the color, whether or not the time is in a respiration period that is appropriate for swallowing, and can further smoothly perform swallowing.

In the swallowing training apparatus according to the present mode, the respiration output part may be configured such that: as the second motion information, the respiration output part causes the display to display strength of the expiration by means of an indicator configured to change in a first direction, and causes the display to display strength of the inspiration by means of an indicator configured to change in a second direction opposite to the first direction. Accordingly, during the training, the subject can clearly and easily understand the state of his/her own respiration, on the basis of the change in the indicator.

Alternatively, the respiration output part may be configured to cause the display to display, as the second motion information, an image in which a respiration waveform is superposed on a straight line separating the expiration and the inspiration from each other. Accordingly, the subject can understand in a simple manner the state of his/her own respiration, together with the shift of the respiration, and can easily perform swallowing so as to meet the trigger for swallowing induction.

In the swallowing training apparatus according to the present mode, the result output part may be configured to cause the display to display a determination result on appropriateness or inappropriateness of the swallowing, together with the first motion information and the second motion information. Accordingly, while the subject conducts the swallowing training with reference to the first motion information and the second motion information displayed on the display, the subject can simultaneously confirm whether or not the timing of the swallowing just performed was ideal, on the basis of the determination result displayed on the display. Accordingly, the subject can smoothly and effectively conduct the swallowing training.

In this case, the swallowing training apparatus may be configured to further include a sound output part configured to output a sound, wherein the result output part causes the sound output part to output a sound indicating the determination result on appropriateness or inappropriateness of the swallowing. Accordingly, the subject can further clearly understand, by the sound, whether or not the timing of the swallowing just performed was ideal.

The swallowing training apparatus according to the present mode may be configured to further include a swallowing output part configured to cause the display to display information indicating occurrence of swallowing, in accordance with the swallowing detection part having detected the swallowing. Accordingly, while the subject understands his/her own respiratory motion with reference to the first motion information and the second motion information displayed on the display, the subject can confirm at which timing in the respiratory motion the subject performed swallowing. Therefore, every time the subject performs swallowing, the subject can confirm whether or not the subject performed swallowing at an ideal timing, and the subject can conduct the swallowing training further effectively.

In the swallowing training apparatus according to the present mode, the swallowing induction part may be configured to provide the trigger to the subject at a predetermined timing in a first half of an expiration period. Accordingly, the swallowing performed by the subject in response to the trigger is more likely to be within the expiration period. Thus, during the training, the subject can easily perform swallowing at an ideal timing with respect to the respiratory motion.

The swallowing induction part may be configured to provide the trigger to the subject by causing a display form of the second motion information in an ideal swallowing period in an expiration period to be different from a display form of a remainder of a respiration period. Accordingly, the subject can understand his/her own respiratory motion on the basis of the second motion information, and can simultaneously receive the trigger for swallowing.

The swallowing training apparatus according to the present mode may be configured to include a sound detection part configured to detect a sound of a larynx portion of the subject, wherein the swallowing detection part detects the swallowing on the basis of respiration information detected by the respiration detection part and sound information detected by the sound detection part. If swallowing is detected with reference to the respiration information together with the sound information, even when a sound similar to a swallowing sound is detected by the sound detection part at a timing when the subject is not performing swallowing, erroneous detection that this similar sound is a sound of swallowing can be inhibited from being performed. Thus, the detection accuracy of swallowing can be increased.

In this case, the swallowing training apparatus may be configured to further include a displacement detection part configured to detect a displacement of the larynx portion of the subject, wherein the swallowing detection part detects the swallowing on the basis of the respiration information detected by the respiration detection part, the sound information detected by the sound detection part, and displacement information detected by the displacement detection part. By detecting swallowing further with reference to the displacement information, it is possible to further increase the detection accuracy of swallowing.

### ADVANTAGEOUS EFFECTS OF THE INVENTION

As described above, according to the present invention, a swallowing training apparatus that enables the subject to effectively conduct swallowing training can be provided.

The effects and the significance of the present invention will be further clarified by the description of the embodiments below. However, the embodiments below are merely examples for implementing the present invention, and the present invention is not limited by the embodiments below in any way.

### BRIEF DESCRIPTION OF THE DRAWINGS

FIG. 1 is a diagram showing an external configuration of a swallowing training apparatus according to an embodiment.
FIG. 2 is a block diagram showing a configuration of the swallowing training apparatus according to the embodiment.
FIG. 3(a) is a flow chart showing an output process according to the embodiment. FIG. 3(b) is a flow chart showing a swallowing detection process according to the embodiment.
FIG. 4(a) is a flow chart showing a determination output process according to the embodiment. FIG. 4(b) is a flow chart showing a swallowing induction process according to the embodiment.
FIG. 5 is a schematic diagram of a respiration waveform which is referred to in description of a method for setting a swallowing induction timing according to the embodiment.
FIG. 6(a) and FIG. 6(b) are each a schematic diagram of a respiration waveform which is referred to in description of another method for setting a swallowing induction timing according to the embodiment.
FIG. 7(a) to FIG. 7(d) are diagrams showing shift of images displayed on a display during swallowing training according to the embodiment.
FIG. 8(a) to FIG. 8(d) are diagrams showing shift of images displayed on the display during swallowing training according to the embodiment.
FIG. 9(a) to FIG. 9(d) are diagrams showing shift of images displayed on the display during swallowing training according to the embodiment.
FIG. 10 is a diagram showing an external configuration of a swallowing training apparatus according to Modification 1.
FIG. 11 is a block diagram showing a configuration of the swallowing training apparatus according to Modification 1.
FIG. 12 is a diagram showing an external configuration of a swallowing training apparatus according to Modification 2.
FIG. 13 is a block diagram showing a configuration of the swallowing training apparatus according to Modification 2.
FIG. 14(a) and FIG. 14(b) are each a diagram showing a configuration of an image displayed on the display according to Modification 3. FIG. 14(c) and FIG. 14(d) are each a diagram showing a configuration of an image displayed on the display according to Modification 4.
FIG. 15 is a flow chart showing a swallowing induction process according to Modification 5.
FIG. 16 is a flow chart showing a determination output process according to Modification 5.
FIG. 17(a) to FIG. 17(d) are each a diagram showing a configuration of an image displayed on the display according to Modification 5.
FIG. 18(a) to FIG. 18(d) are each a diagram showing a configuration of an image displayed on the display according to Modification 6.

It should be noted that the drawings are solely for description and do not limit the scope of the present invention by any degree.

### BEST MODE FOR CARRYING OUT THE INVENTION

The present embodiment is obtained by applying the present invention to a swallowing training apparatus for conducting training such that swallowing is performed at an ideal timing with respect to respiration. In general, aspiration easily occurs if inspiration is performed either immediately before swallowing or immediately after swallowing. Therefore, an ideal timing of swallowing with respect to respiration is a timing in an expiration period that includes both of a timing immediately before the swallowing and a timing immediately after the swallowing. A swallowing training apparatus according to the present embodiment enables a subject to conduct training so as be able to perform swallowing at a timing in an expiration period that includes both of a timing immediately before the swallowing and a timing immediately after the swallowing".

Hereinafter, a swallowing training apparatus 100 according to the present embodiment is described with reference to the drawings.

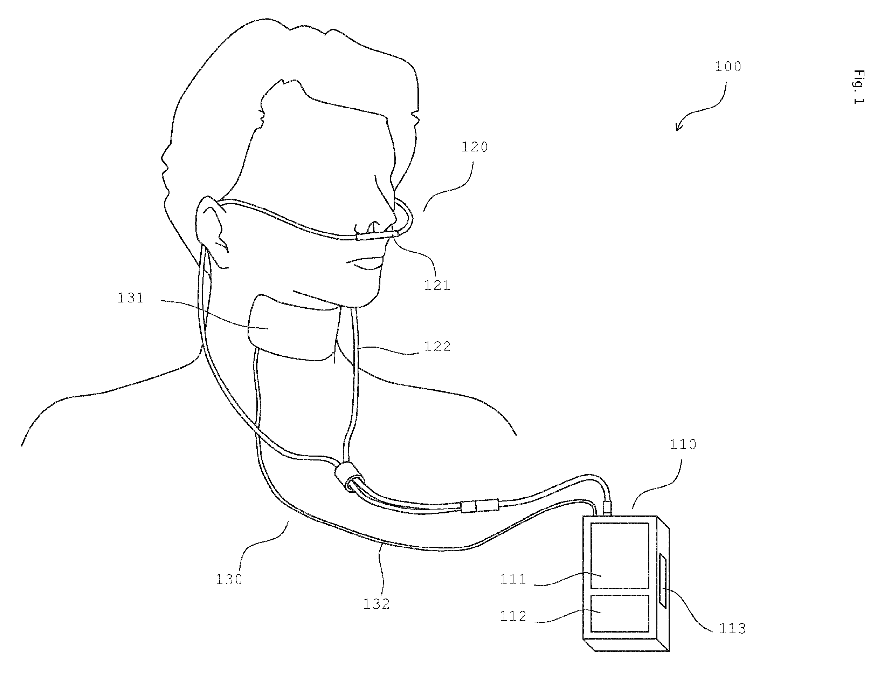

FIG. 1 is a diagram showing an external configuration of the swallowing training apparatus 100 according to the present embodiment.

The swallowing training apparatus 100 includes a terminal device 110, a nasal cannula 120, and a detection part 130.

The terminal device 110 includes a display 111 and an input part 112. The terminal device 110 is configured to be small in size and light in weight so as to be easily carried by a subject. The subject inputs an instruction to the terminal device 110 through the input part 112 while confirming the display on the display 111. The input part 112 includes a button, a key, and the like.

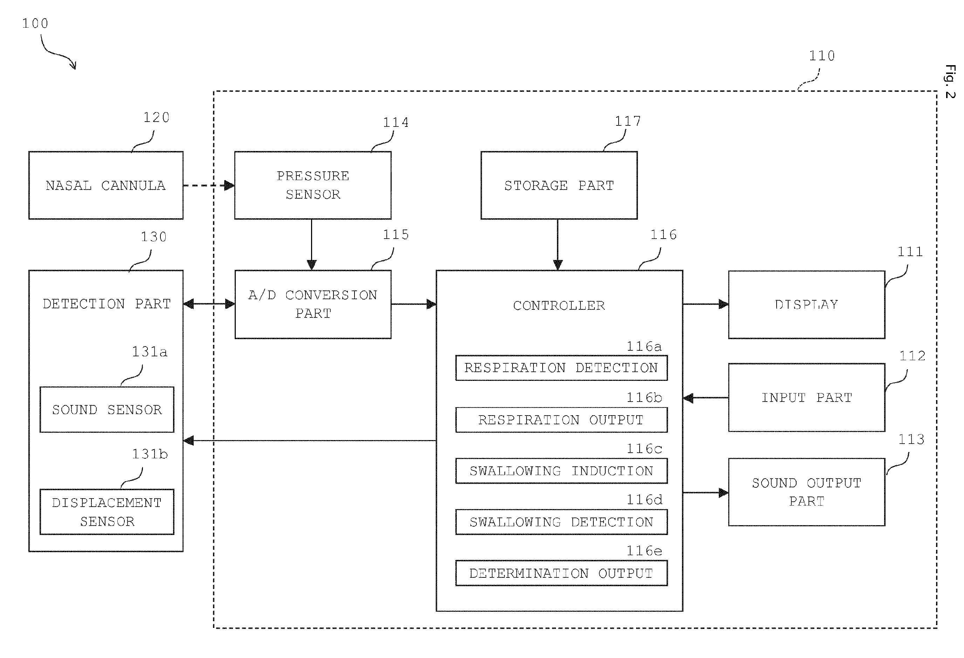

The nasal cannula 120 includes: an attachment part 121 having a pair of tubular members; and a tube 122 connected to both ends of the attachment part 121. The pair of tubular members of the attachment part 121 are inserted into the nasal cavities of the subject, and the other end of the tube 122 is connected to the terminal device 110. Accordingly, when the subject performs respiration, the air in the tube 122 flows, and the flow of the air in the tube 122 is detected as a pressure by a pressure sensor 114 (see FIG. 2) in the terminal device 110. Even when the subject is breathing through the mouth, since the nasal cavities and the oral cavity are connected to each other, the air in the tube 122 flows and the pressure changes.

The detection part 130 includes a pad 131 which is thin and flexible, and a cable 132. The pad 131 is attached to a larynx portion of the subject. The pad 131 includes a sound sensor 131a (see FIG. 2) for detecting a sound of the larynx portion; and a displacement sensor 131b (see FIG. 2) for detecting, in terms of pressure, a displacement of the hyoid bone in accordance with deformation of the larynx portion.

FIG. 2 is a block diagram showing a configuration of the swallowing training apparatus 100.

The terminal device 110 includes a sound output part 113, the pressure sensor 114, an A/D conversion part 115, a controller 116, and a storage part 117, in addition to the display 111 and the input part 112 shown in FIG. 1.

The sound output part 113 includes a buzzer and a speaker, and outputs to outside a predetermined sound on the basis of control by the controller 116.

The pressure sensor 114 detects, as a pressure, the flow of air guided by the tube 122 of the nasal cannula 120, and outputs the detected analog pressure signal to the A/D conversion part 115. The detection part 130 includes the sound sensor 131a and the displacement sensor 131b. The sound sensor 131a detects a sound in the vicinity of the larynx portion of the subject, and outputs the detected analog sound signal to the A/D conversion part 115. The displacement sensor 131b detects, as a displacement of the hyoid bone, deformation of the larynx portion of the subject, and outputs the detected analog displacement signal to the A/D conversion part 115.

The A/D conversion part 115 performs sampling, in a predetermined cycle, the pressure signal, the sound signal, and the displacement signal respectively outputted from the pressure sensor 114, the sound sensor 131a, and the displacement sensor 131b, and outputs pieces of digital data that correspond to the respective sampling signals, to the controller 116. Hereinafter, the respective pieces of digital data obtained through A/D conversion of the pressure signal, the sound signals, and the displacement signal will be referred to as pressure data, sound data, and displacement data.

The controller 116 controls components of the terminal device 110 in accordance with a program stored in the storage part 117. The controller 116 is provided with functions of a respiration detection part 116a, a respiration output part 116b, a swallowing induction part 116c, a swallowing detection part 116d, and a determination output part 116e, by the program stored in the storage part 117.

The respiration detection part 116a performs processing such as noise reduction on the pressure data inputted from the A/D conversion part 115, and obtains respiration data that reflects the respiratory motion of the subject. The respiration data is sequentially stored into the storage part 117.

On the basis of the respiration data obtained by the respiration detection part 116a, the respiration output part 116b causes the display 111 to display: first motion information which indicates which of expiration and inspiration the current respiratory motion belongs to; and second motion information which indicates the progress state of expiration and inspiration.

On the basis of the respiration data obtained by the respiration detection part 116a, the swallowing induction part 116c causes the sound output part 113 to output a sound (trigger) that prompts the subject to perform swallowing, at an ideal swallowing timing with respect to the respiratory motion.

On the basis of the respiration data obtained by the respiration detection part 116a and the sound data and the displacement data inputted from the A/D conversion part 115, the swallowing detection part 116d detects swallowing performed by the subject, and causes the display 111 to display information indicating that the swallowing was performed.

The determination output part 116e determines whether or not the swallowing detected by the swallowing detection part 116d was at an ideal timing with respect to the respiratory motion based on the respiration data. Further, the determination output part 116e causes the display 111 to display a determination result as an image, and causes the sound output part 113 to output the determination result as a sound.

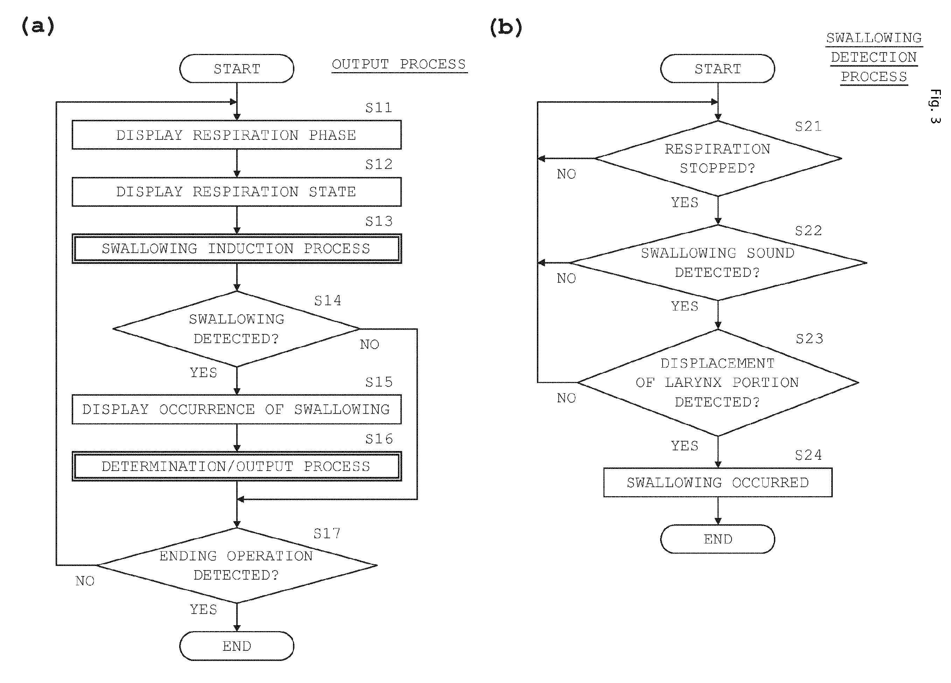

FIG. 3(a) is a flow chart showing an output process for outputting various types of information to outside during operation of the swallowing training apparatus 100.

When operation of the swallowing training apparatus 100 has started, the processes of S11 and S12 are performed by the respiration output part 116b. In S11, on the basis of the respiration data obtained by the respiration detection part 116a the respiration output part 116b causes the display 111 to display information (the first motion information) indicating which of expiration and inspiration the respiratory motion at the current time point belongs to. In S12, on the basis of the respiration data, the respiration output part 116b causes the display 111 to display information indicating the progress state of expiration and inspiration (the second motion information) (S12) .

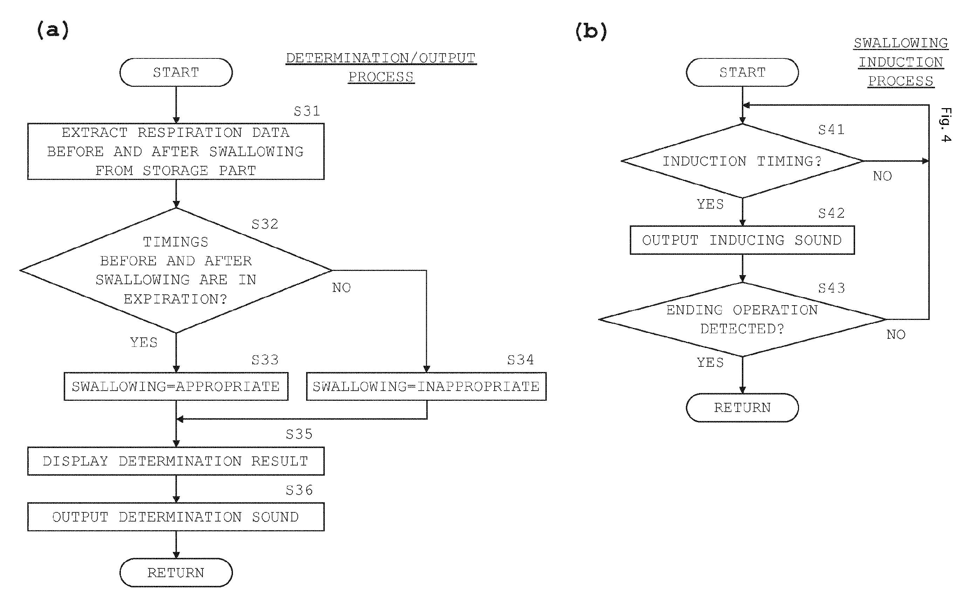

In parallel to the processes of S11 and S12, a swallowing induction process of S13 is performed by the swallowing induction part 116c. Through this process, at a predetermined timing in the respiratory motion, a trigger that prompts swallowing is provided to the subject. The swallowing induction process (S13) will be described later with reference to FIG. 4(b), FIG. 5, FIG. 6(a), and FIG. 6(b).

The processes of S11 to S13 are repeatedly performed until operation of the swallowing training apparatus 100 ends (S17: YES). During the time, the processes of S14 and S15 are performed by the swallowing detection part 116d. In S14, the swallowing detection part 116d determines whether or not swallowing has been detected. When swallowing has been detected (S14: YES), the swallowing detection part 116d causes, in S15, the display 111 to display information indicating occurrence of swallowing. The swallowing detection process (S14) will be described later with reference to FIG. 3(b).

Further, in accordance with the detection of the swallowing, a determination output process of S16 is performed by the determination output part 116e. In S16, the determination output part 116e determines appropriateness or inappropriateness of the detected swallowing, and outputs a determination result through the display 111 and the sound output part 113 to outside. The determination output process (S16) will be described later with reference to FIG. 4(a).

FIG. 3(b) is a flow chart showing the swallowing detection process (S14 in FIG. 4(a)).

In S21, with reference to the respiration data, the swallowing detection part 116d determines whether or not respiration of the subject stopped for a certain period or longer (for example, 400 msec or longer). When the determination in S21 is YES, the swallowing detection part 116d determines, in S22, whether or not a sound that corresponds to a swallowing sound has been detected in the apneic period determined in S21, on the basis of the sound data outputted from the A/D conversion part 115. Further, in S23, on the basis of the displacement data outputted from the A/D conversion part 115, the swallowing detection part 116d determines whether or not a displacement of the hyoid bone that corresponds to a deformation of the larynx portion at the time of swallowing has been detected in the apneic period determined in S21. Only when all the determinations in S21 to S23 are YES, the swallowing detection part 116d determines, in S24, that swallowing occurred.

In general, at the time of swallowing, respiration stops. Therefore, if the determination in S21 is included in the condition of occurrence of swallowing, detection accuracy of swallowing can be increased. For example, in S22, the sound data is subjected to frequency analysis, and on the basis of intermittency and continuity of the sound, it is determined whether or not the sound based on the sound data corresponds to a swallowing sound. Thus, by using intermittency and continuity of sound as parameters to determine the presence or absence of swallowing, it is possible to increase the detection accuracy of swallowing. It should be noted that the determination in S23 may be omitted. In this case, the displacement sensor 131b is excluded from the detection part 130.

FIG. 4(a) is a flow chart showing the determination output process (S16 in FIG. 3(a)).

In S31, the determination output part 116e extracts, from the storage part 117, respiration data that corresponds to a predetermined period before and after the swallowing. In S32, the determination output part 116e determines whether or not both of a timing immediately before and a timing immediately after the swallowing detected by the swallowing detection part 116d are included in an expiration period. When the determination in S32 is YES, the determination output part 116e obtains, in S33, a determination result that the swallowing was performed at an ideal timing with respect to the respiration. When the determination in S32 is NO, the determination output part 116e obtains, in S34, a determination result that the swallowing was not performed at an ideal timing with respect to the respiration. In S35, the determination output part 116e causes the display 111 to display information based on the obtained determination result. Further, in S36, the determination output part 116e causes the sound output part 113 to output a sound based on the obtained determination result.

FIG. 4(b) is a flow chart showing the swallowing induction process (S13 in FIG. 3(a)).

In S41, with reference to the respiration data, the swallowing induction part 116c determines whether or not the swallowing induction timing has arrived. When the determination in S41 is YES, the swallowing induction part 116c causes, in S42, the sound output part 113 to output a sound for prompting the subject to perform swallowing. The swallowing induction part 116c repeats the processes of S41 and S43 until an ending operation of the training operation is detected in S43.

When a swallowing induction timing is to be determined, it is necessary to take into consideration the time period from when the subject has performed swallowing in response to the induction until the subject starts inspiration. That is, even when swallowing has been induced in an expiration period, if the time period from when the subject has performed swallowing in response to the induction until the subject starts inspiration is short, it is highly likely that the swallowing and the inspiration occur consecutively, which increases the possibility of occurrence of aspiration. Therefore, when swallowing is to be induced, it is necessary to set the swallowing induction timing such that the time period from swallowing to the start of inspiration is not short.

For example, in a case where swallowing is induced in the subject in the middle or in the second half of an expiration period, there is an interval from when the subject has recognized the inducing sound until the subject actually performs swallowing. In the case of a subject who needs swallowing training, it is considered that this interval tends to be long, and in particular, the older the subject is, the longer this interval becomes.

Therefore, preferably, the timing of swallowing induction is in the first half of an expiration period. In the present embodiment, an inducing sound is outputted from the sound output part 113 at a predetermined timing in the first half of an expiration period.

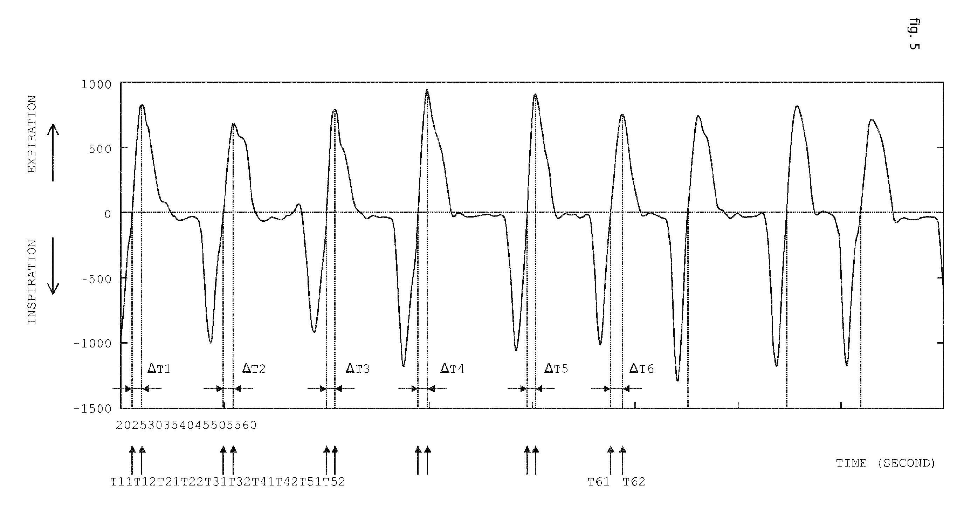

FIG. 5 is a diagram describing a method for setting a swallowing induction timing. In this setting method, the timing when expiration in the first half of an expiration period peaks is set as the swallowing induction timing.

FIG. 5 shows a graph of a respiration waveform based on respiration data. The vertical axis represents pressure and the horizontal axis represents time. T11, T21, T31, T41, T51, and T61 are timings at each of which expiration is started. T12, T22, T32, T42, T52, and T62 are timings at each of which expiration peaks. Each of ΔT1 to ΔT6 is the difference between the timing when expiration is started and the timing when expiration peaks.

In the induction timing setting, first, when start of expiration has been detected, an average value ΔT of the difference between this time point when the start of expiration has been detected and the timing when the expiration peaks, is estimated on the basis of differences ΔTn of five expirations preceding that time point. The difference Tn is sequentially stored into the storage part 117.

For example, as shown in FIG. 5, when the expiration start time T61 has been obtained, an average value ΔT of differences from this timing is obtained by averaging the differences ΔT1 to ΔT5. Then, the timing when time has elapsed by the average value ΔT from the expiration start time T61 is estimated as the timing when the expiration peaks, and at this timing, an inducing sound for inducing swallowing is outputted. Thereafter, in a similar manner, when an expiration start time has been obtained, an inducing sound is outputted at the timing when time has elapsed by an average value ΔT of differences ΔTn of five expirations preceding that time point of the obtainment of the expiration start time. In this manner, the swallowing induction timing is set to a timing in the first half of an expiration period, and the inducing sound is outputted.

The range of expirations to be averaged is not limited to five expirations preceding the expiration at the current time point, and may be set to another range, such as ten expirations preceding the expiration at the current time point. The method for obtaining ΔT is not limited to averaging, and another method may be used.

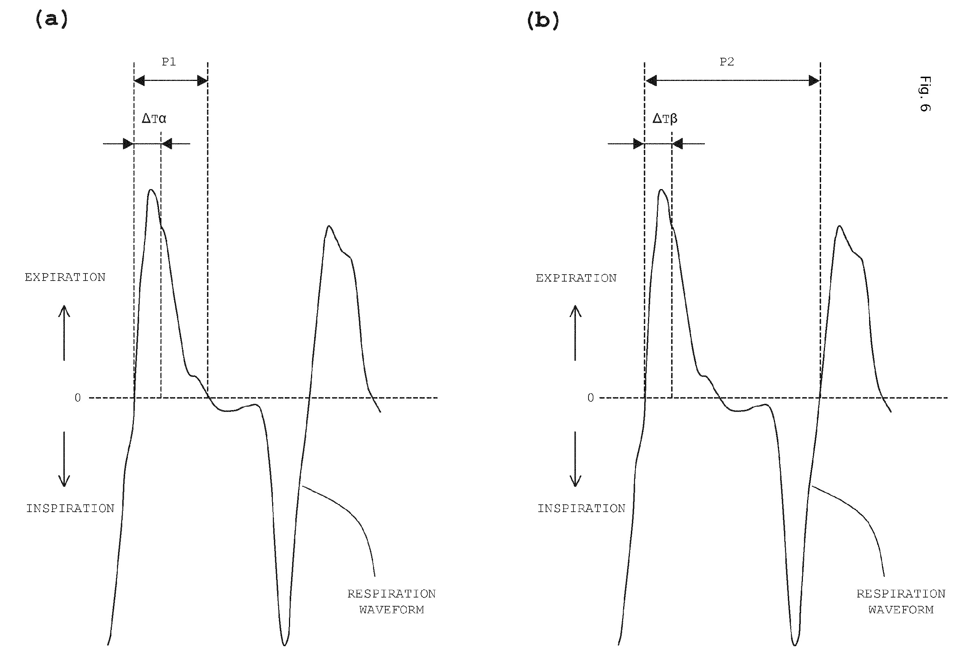

Each of FIG. 6(a) and FIG. 6(b) is a diagram describing another method for setting the swallowing induction timing.

In FIG. 6(a), the timing when a time period ΔTα corresponding to a predetermined proportion α (for example, 1/3) of an expiration period P1 has elapsed from the expiration start time point is set as the swallowing induction timing. Also in this case, similar to the case shown in FIG. 5, an average value of time periods ΔTαn obtained from five expirations preceding the expiration at the current time point is obtained as the time period ΔTα for the expiration at the current time point.

In FIG. 6(b), the timing when a time period ΔTβ corresponding to a predetermined proportion β (for example, 1/8) of a respiration period P2 has elapsed from the expiration start time point is set as the swallowing induction timing. Also in this case, similar to the case shown in FIG. 5, an average value of time periods ΔTβn obtained from five respirations preceding the respiration at the current time point is obtained as the time period ΔTβ for the respiration at the current time point.

As shown in FIG. 6(a) and FIG. 6(b), in a case where the time period ΔTα and the time period ΔTβ are determined, the proportion α, β is set such that the timing when the time period ΔTα, ΔTβ has elapsed from the expiration start is included in the first half of the expiration period.

In the setting method shown in FIG. 5, the timing when the expiration peaks is estimated on the basis of past differences ΔTn. However, instead of this, at the timing when the peak of the expiration is detected on the basis of expiration data, the swallowing sound may be outputted. Other than this, immediately after expiration is started, an inducing sound for inducing swallowing may be outputted.

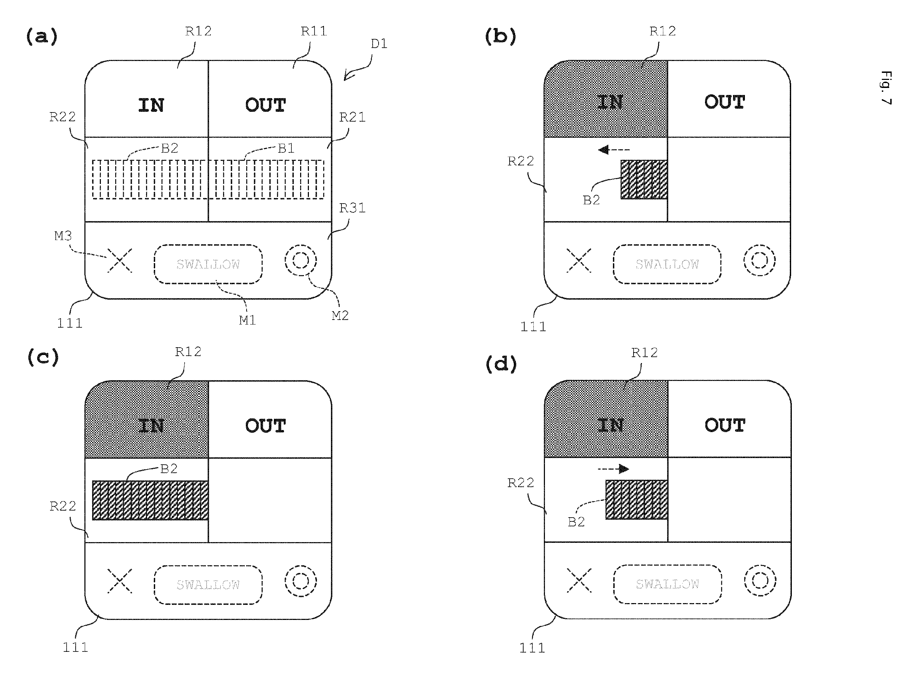

FIG. 7(a) to FIG. 9(d) show shift of images displayed on the display 111 through the output process shown in FIG. 3(a).

First, with reference to FIG. 7(a), a configuration of a screen D1 displayed on the display 111 is described.

The screen D1 includes: regions R11, R12 indicating which of expiration and inspiration the respiratory motion is in; regions R21, R22 indicating the progress state of expiration and inspiration; and a region R31 for displaying occurrence of swallowing and appropriateness or inappropriateness of the swallowing. The first motion information described above is displayed in the region R11, R12. The second motion information described above is displayed in the region R21, R22.

Characters "OUT" indicating that the respiratory motion is expiration are shown in the region R11, and characters "IN" indicating that the respiratory motion is inspiration are shown in the region R12. When the respiratory motion is expiration, the background of the region R11 is displayed in a color indicating that the time is appropriate for swallowing, and when the respiratory motion is inspiration, the background of the region R12 is displayed in a color indicating that the time is not appropriate for swallowing. For example, the display color of the background of the region R11 is set to green, and the display color of the background of the region R12 is set to red. The display color of the background of the region R11 may be blue.

When the respiratory motion is expiration, an identifier (bar meter B1) which indicates the strength of the expiration is displayed in the region R21. When the respiratory motion is inspiration, an identifier (bar meter B2) which indicates the strength of the inspiration is displayed in the region R22. The bar meter B1 is displayed so as to stepwise extend in the right direction in accordance with the strength of the expiration from the boundary between the regions R21 and R22. The bar meter B2 is displayed so as to stepwise extend in the left direction in accordance with the strength of the inspiration from the boundary between the regions R21 and R22. The bar meter B1, B2 is composed of a plurality of segments arranged in the left-right direction, and the bar meter B1, B2 expands and contracts through control of the lighting of each segment. For convenience, in FIG. 7(a), the segments are indicated by broken lines in the region R21, R22.

In the region R31, when swallowing has been detected, a mark M1 for indicating occurrence of swallowing is displayed. In the region R31, a mark M2 is displayed when the timing of the detected swallowing is ideal with respect to the respiration, and a mark M3 is displayed when the timing of the detected swallowing is not ideal with respect to the respiration.

As shown in FIG. 7(b), when the training has been started and the subject performs inspiration, the background of the region R12 is displayed in red, and the bar meter B2 is displayed in the region R22. In accordance with the strength of the inspiration, the bar meter B2 expands in the direction of the broken line arrow, and when the strength of the inspiration becomes maximum, the state shown in FIG. 7(c) is established. Then, in accordance with decrease from the maximum of the strength of the inspiration, the bar meter B2 contracts in the direction of the broken line arrow, as shown in FIG. 7(d).

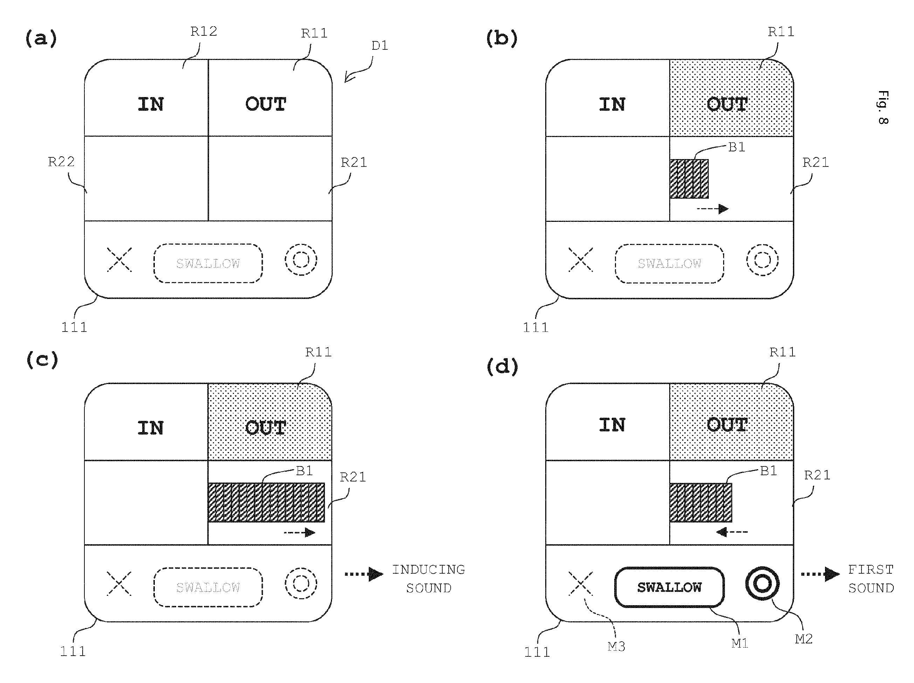

Then, at the timing when the respiratory motion is switched from inspiration to expiration, the screen D1 enters the state shown in FIG. 8(a). Then, as shown in FIG. 8(b), when the expiration advances, the background of the region R11 is displayed in green, and the bar meter B1 is displayed in the region R21. In accordance with the strength of the expiration, the bar meter B1 expands in the direction of the broken line arrow, and when the strength of the expiration becomes maximum, the state shown in FIG. 8(c) is established. Around this timing, an inducing sound for prompting the subject to perform swallowing is outputted.

When the subject has performed swallowing in response to the inducing sound, the mark M1 indicating occurrence of swallowing is displayed, first, as shown in FIG. 8(d). Then, when appropriateness or inappropriateness of the swallowing has been determined through the process shown in FIG. 4(a), the mark M2 indicating a determination result is displayed. Here, since both of the timing immediately before the swallowing and the timing immediately after the swallowing are included in the expiration period, the mark M2 indicating that the timing of the swallowing was ideal is displayed. At this time, simultaneously, a sound indicating that the swallowing was ideal (first sound) is outputted.

When the period from the output of the inducing sound to the occurrence of swallowing has become long, and one or both of the timing immediately before the swallowing and the timing immediately after the swallowing have failed to be included in the expiration period, the mark M3 indicating that the timing of the swallowing was not ideal is displayed instead of the mark M2. At this time, simultaneously, a sound indicating that the swallowing was not ideal (second sound) is outputted.

The display of the mark M1, M2, M3 and the output of the inducing sound, and the first sound or the second sound, are performed only for a predetermined time period (for example, about 1 second) that allows the subject to confirm such display and output.

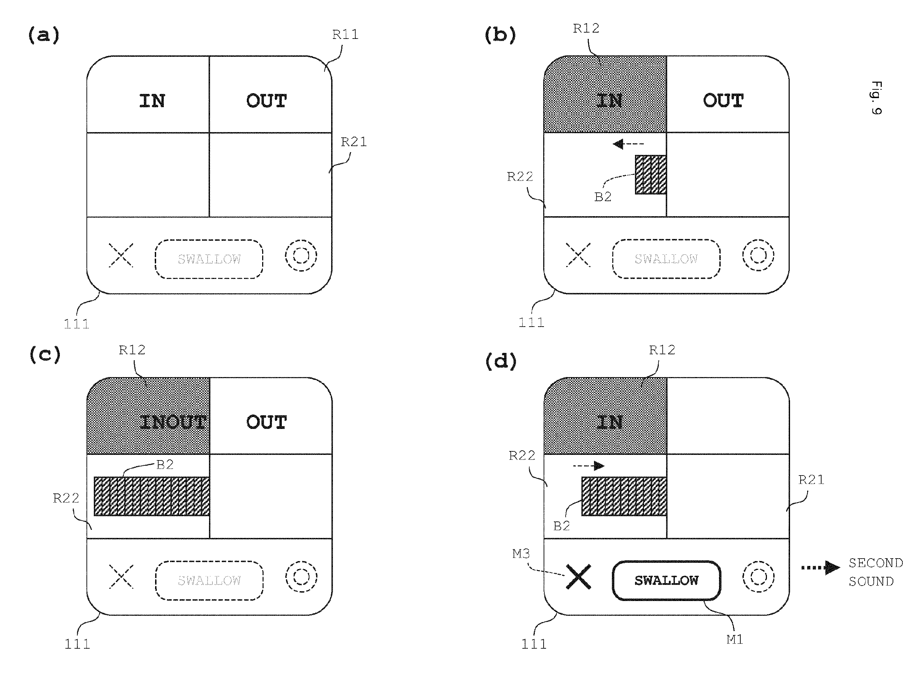

Then, at the timing when the respiratory motion is switched from expiration to inspiration, the screen D1 enters the state shown in FIG. 9(a). Thereafter, as shown in FIG. 9(b), when the inspiration advances, the background of the region R12 is displayed in red and the bar meter B2 is displayed in the region R22, again. In accordance with the strength of the inspiration, the bar meter B2 expands in the direction of the broken line arrow, and when the strength of the inspiration becomes maximum, the state shown in FIG. 9(c) is established.

If the subject has performed swallowing by mistake around this timing, the mark M1 indicating occurrence of swallowing is displayed, first, as shown in FIG. 9(d), and further, appropriateness or inappropriateness of the swallowing is determined by the process shown in FIG. 4(a). Here, since both of the timing immediately before the swallowing and the timing immediately after the swallowing are included in the inspiration period, it is determined that the swallowing timing was not ideal, and the mark M3 is displayed. At this time, simultaneously, a sound indicating that the swallowing was not ideal (the second sound) is outputted.

Thereafter, the swallowing training to the subject is conducted in a similar manner. When an input for ending the swallowing training is performed, the process shown in FIG. 3(a) ends, and the displaying on the display 111 ends.

### <Effects of the embodiment>

According to the present embodiment, the following effects can be exhibited.

For example, as shown in FIG. 8(d), every time swallowing is performed by the subject in response to the inducing sound (trigger), information (the mark M2, M3) indicating whether or not the swallowing was ideal is displayed on the display 111. Thus, the subject can confirm, whenever necessary during the swallowing training, whether or not the subject performed the swallowing at an ideal timing. In addition, the speech-language-hearing therapist or the like also can confirm whenever necessary whether or not the subject performed the swallowing at an ideal timing in a respiratory motion. Therefore, the subject can conduct the swallowing training smoothly and effectively by himself/herself or while being instructed by the speech-language-hearing therapist or the like.

As shown in FIG. 7(a) to FIG. 9(d), information indicating the respiratory motion of the subject is displayed in the region R11, R12 and the region R21, R22. Accordingly, on the basis of the information displayed in these regions, the subject can be clearly conscious of the respiratory motion, which is difficult for the subject to be conscious of. Thus, during the training, the subject can perform swallowing so as to be at an ideal timing with respect to the respiratory motion, while being clearly conscious of his/her own respiratory motion.

As shown in FIG. 7(a) to FIG. 9(d), the first motion information indicating which of expiration and inspiration the respiratory motion is in is displayed in the region R11, R12, and the second motion information indicating the progress state of expiration and inspiration is displayed in the region R21, R22. Accordingly, with reference to the display 111, the subject can understand which of expiration and inspiration his/her own respiration is in, and can understand the progress state of expiration and inspiration at each time point. Therefore, the subject can smoothly perform swallowing at an ideal timing in the respiratory motion, in response to the swallowing inducing sound (trigger).

As described with reference to FIG. 7(a) to FIG. 9(d), the background color of the region R11 and the background color of the region R12 are set to be different from each other. Accordingly, the subject can intuitively understand, by the color, which of expiration and inspiration the respiratory motion at the current time point is in, and can more smoothly perform swallowing in response to the inducing sound (trigger).

Further, in the present embodiment, the background color of the region R11 indicating expiration is set to a color (green) indicating that the time is appropriate for swallowing, and the background color of the region R12 indicating inspiration is set to a color (red) indicating that the time is not appropriate for swallowing. Accordingly, the subject can intuitively understand, by the color, whether or not the current time point is in a respiration period that is appropriate for swallowing, and can further smoothly perform swallowing.

As shown in FIG. 7(a) to FIG. 9(d), the bar meter B1 indicating the strength of expiration is displayed in the region R21, and the bar meter B2 indicating the strength of inspiration is displayed in the region R22. Accordingly, during the training, the subject can clearly and easily understand the state and the rhythm of his/her own respiration, on the basis of the change in the bar meter B1, B2. Thus, the subject can perform swallowing so as to be within the expiration period, in response to the inducing sound (trigger).

As shown in FIG. 9(d), the mark M2, M3 indicating a determination result on appropriateness or inappropriateness of the swallowing is displayed in the display 111, together with the first motion information displayed in the region R11, R12 and the second motion information displayed in the region R21, R22. Accordingly, while the subject is conducting swallowing training with reference to the first motion information and the second motion information displayed on the display 111, the subject can simultaneously confirm whether or not the timing of the swallowing just performed was ideal, on the basis of the mark M2, M3 displayed on the display 111. Thus, the subject can smoothly and effectively conduct the swallowing training.

Further, in the present embodiment, a sound indicating the determination result on appropriateness or inappropriateness of the swallowing (the first sound, the second sound) is outputted. Accordingly, the subject can further clearly understand, by the sound, whether or not the timing of the swallowing just performed was ideal.

As shown in FIG. 8(d), in accordance with the detection of the swallowing, the mark M1 that indicates occurrence of swallowing is displayed on the display 111. Accordingly, while the subject understands his/her own respiratory motion with reference to the first motion information and the second motion information displayed on the display 111, the subject can confirm at which timing in the respiratory motion the subject performed the swallowing. Therefore, every time the subject performs swallowing, the subject can confirm whether or not the subject performed the swallowing at an ideal timing, and the subject can conduct the swallowing training further effectively.

As described with reference to FIG. 5, FIG. 6(a), and FIG. 6(b), an inducing sound (trigger) is outputted at a predetermined timing in the first half of an expiration period. Accordingly, the swallowing performed by the subject in response to the inducing sound (trigger) is more likely to be within the expiration period. Thus, during the training, the subject can easily perform swallowing at an ideal timing with respect to the respiratory motion.

As shown in FIG. 3(b), swallowing is detected on the basis of the respiration data obtained by the respiration detection part 116a, and the sound data detected by the sound sensor 131a. Accordingly, even when a sound similar to a swallowing sound is detected by the sound sensor 131a at a timing when the subject is not performing swallowing, erroneous detection that this similar sound is a sound of swallowing can be inhibited from being performed. Thus, the detection accuracy of swallowing can be increased.

Further, in the present embodiment, swallowing is detected also on the basis of the displacement data detected by the displacement sensor 131b which detects a displacement of the larynx portion of the subject. Therefore, the detection accuracy of swallowing can be further increased.

In the present embodiment, notification of appropriateness or inappropriateness of swallowing is made in terms of both of the display (the mark M2, M3) and the sound (the first sound, the second sound). However, notification of appropriateness or inappropriateness of swallowing may be made in terms of either one of the display and the sound. The respiratory motion of the subject may be indicated by either one of the display of the background color in the region R11, R12 and the display of the bar meter B1, B2 in the region R21 R22. Further, a sound that indicates occurrence of swallowing may be outputted in accordance with detection of the swallowing.

Other than these, the embodiment of the present invention can be modified in various manners as below.

### <Modification 1>

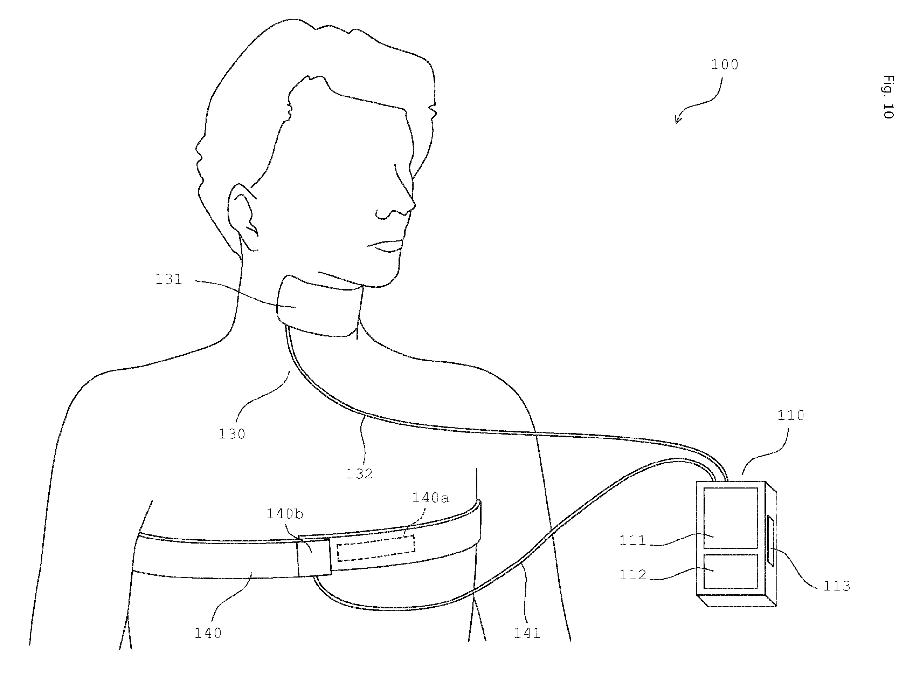

FIG. 10 is a diagram showing an external configuration of a swallowing training apparatus 100 according to Modification 1.

In Modification 1, a detection band 140 is used instead of the nasal cannula 120. The detection band 140 is formed from a stretchable belt-shaped member, and includes a stretchable strain sensor 140a therein. A detection signal from the strain sensor 140a is supplied to the terminal device 110 through a cable 141. A fastener 140b such as a hook-and-loop fastener is provided at both ends of the detection band 140. In a state where the detection band 140 is wound on the chest of the subject, both ends of the detection band 140 are joined by the fastener 140b, whereby the detection band 140 is attached to the subject. When the subject performs respiration, the strain sensor 140a stretches and contracts in association with the respiration, and a detection signal in accordance with the stretch or contraction is supplied to the terminal device 110. On the basis of this detection signal, respiration of the subject is detected.

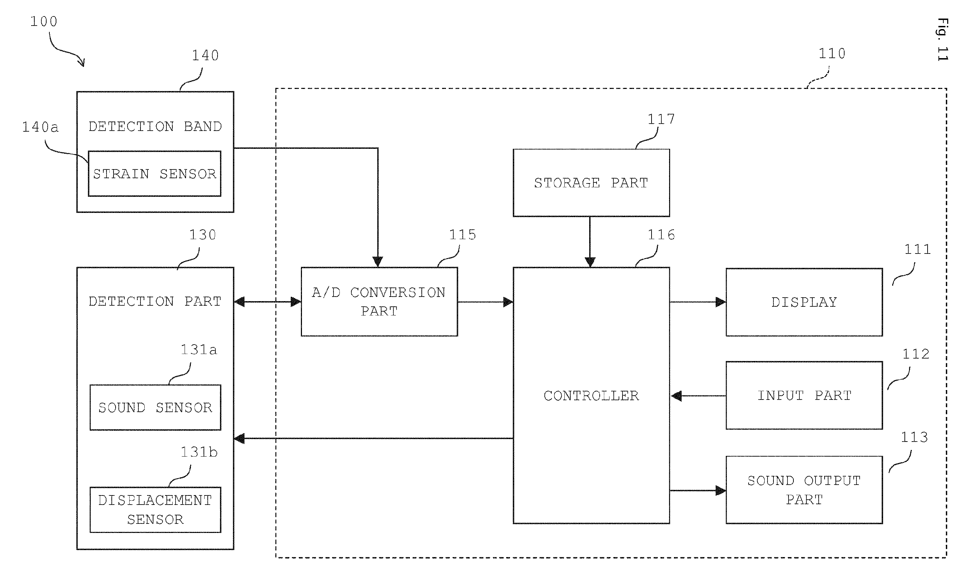

FIG. 11 is a block diagram showing a configuration of the swallowing training apparatus 100 according to Modification 1.

In Modification 1, the detection signal from the strain sensor 140a is inputted to the A/D conversion part 115. The A/D conversion part 115 converts the detection signal inputted from the strain sensor 140a into digital data. Similar to the case where the nasal cannula 120 is used in the above embodiment, the converted digital data serves as respiration data that reflects the respiration of the subject. On the basis of the thus-generated respiration data, processes, displaying, and sound output similar to those in the above embodiment are performed.

Therefore, also in Modification 1, effects similar to those in the above embodiment can be exhibited. In addition, in Modification 1, instead of the nasal cannula 120, the detection band 140 is attached to the subject, and thus, the subject can perform a respiratory motion more smoothly during the swallowing training, and discomfort felt by the subject during the swallowing training can be alleviated. Therefore, the subject can conduct the swallowing training more smoothly and comfortably.

### <Modification 2>

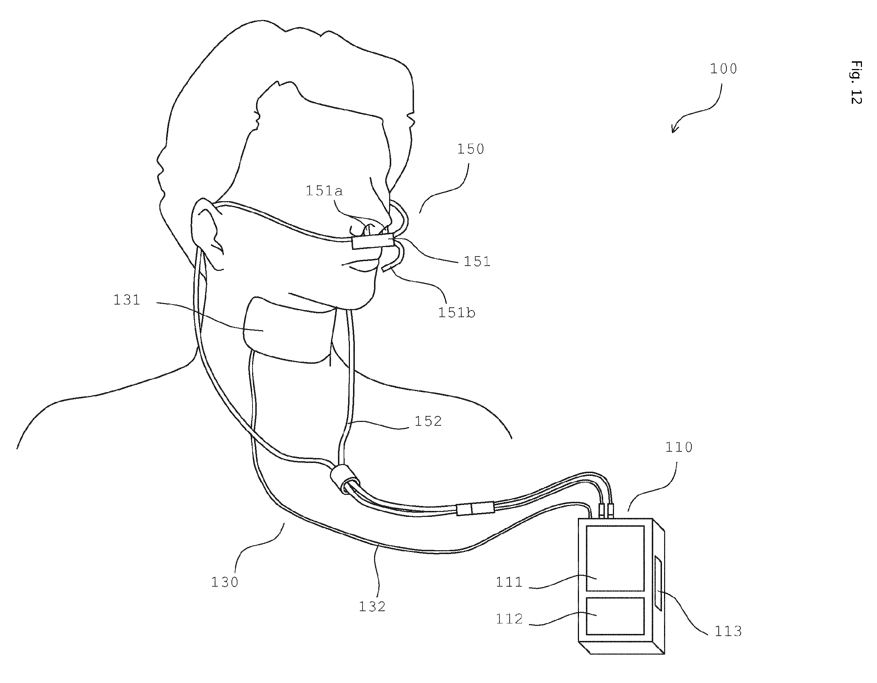

FIG. 12 is a diagram showing an external configuration of a swallowing training apparatus 100 according to Modification 2.

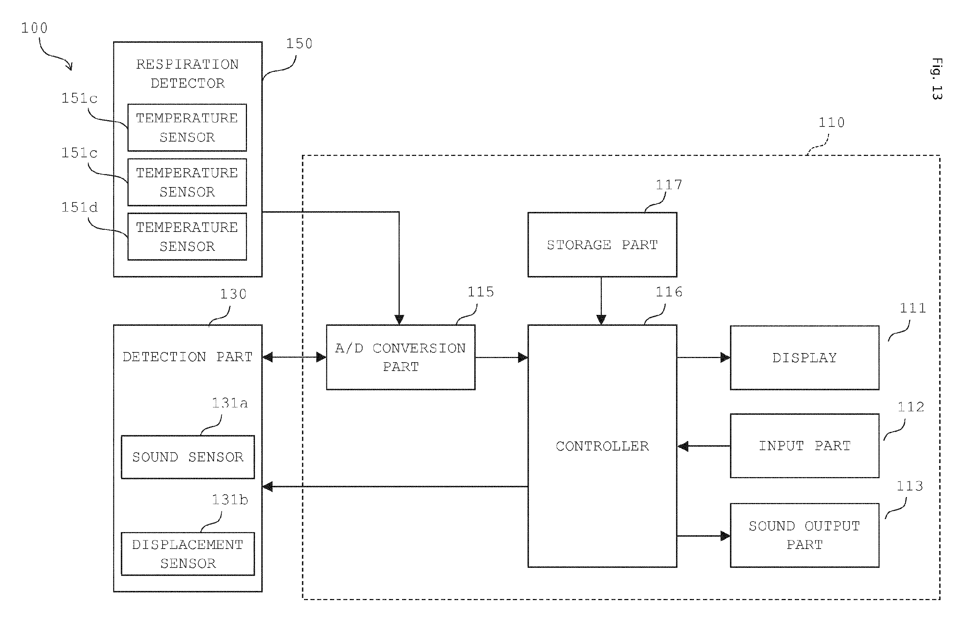

In Modification 2, a thermistor-type respiration detector 150 is used instead of the nasal cannula 120. The respiration detector 150 includes a detection part 151 and a cable 152. The detection part 151 includes: two bar-shaped support portions 151a configured to be respectively inserted into two nostrils; and a bar-shaped support portion 151b extending toward the mouth of the subject. Temperature sensors 151c (see FIG. 13) are respectively provided at the tips of the two support portions 151a, and a temperature sensor 151d (see FIG. 13) is also provided at the tip of the support portion 151b. Detection signals from the temperature sensors 151c, 151d are supplied to the terminal device 110 through the cable 152. The respiration detector 150 is attached to the subject in a similar manner to that of the nasal cannula 120.

When the subject performs respiration, detection signals according to expiration and inspiration are supplied to the terminal device 110. When the subject performs respiration, in an expiration period, the temperature detected by each temperature sensor 151c or the temperature sensor 151d increases due to the breath released from inside of the body; and in an inspiration period, the temperature detected by each temperature sensor 151c or the temperature sensor 151d decreases due to the air taken into the body from outside. This temperature change is reflected in the detection signals from the temperature sensors 151c and the temperature sensor 151d. On the basis of the detection signals, respiration of the subject is detected.

FIG. 13 is a block diagram showing a configuration of the swallowing training apparatus 100 according to Modification 2.

In Modification 2, detection signals from the temperature sensors 151c, 151d are inputted to the A/D conversion part 115. The A/D conversion part 115 converts the detection signals inputted from the temperature sensors 151c, 151d into digital data. The converted digital data serves as respiration data that reflects the respiration of the subject, similar to the case where the nasal cannula 120 of the above embodiment is used. In Modification 1, on the basis of the thus-generated respiration data, processes, displaying, and sound output similar to those in the above embodiment are performed. Therefore, also in Modification 1, effects similar to those in the above embodiment can be exhibited.

### <Modification 3>

In the above embodiment, in the displays on the screen D1 shown in FIG. 7(a) to FIG. 9(d), the colors of the bar meters B1, B2 displayed in the regions R21, R22 are set to the same color. However, the colors of the bar meters B1, B2 may be different from each other, as in the case of the regions R11, R12.

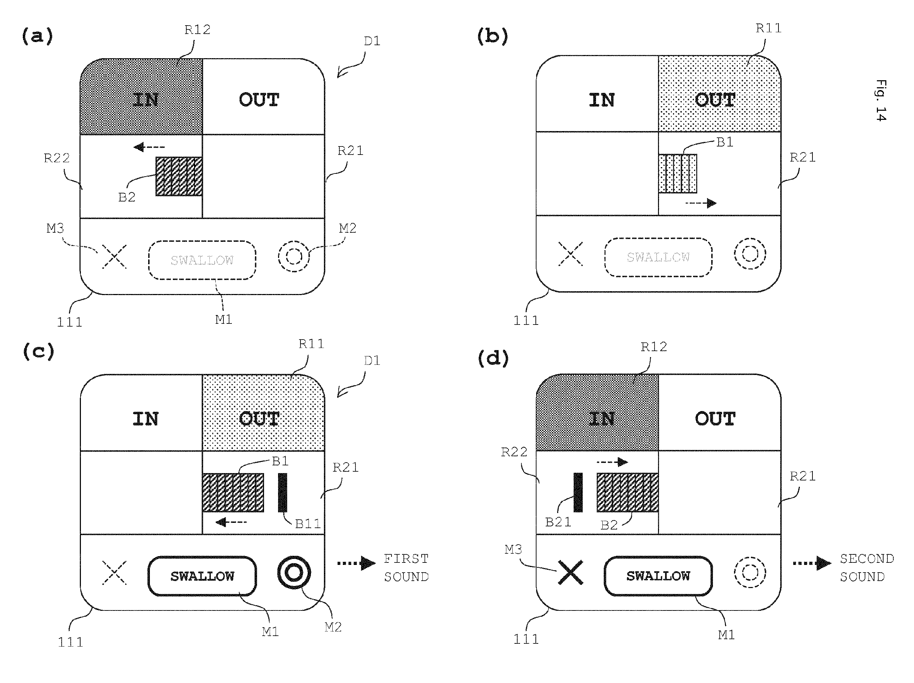

Each of FIG. 14(a) and FIG. 14(b) is a diagram showing a configuration of the screen D1 displayed on the display 111 according to Modification 3. As shown in FIG. 14(a) and FIG. 14(b), in Modification 3, the color of the bar meter B1 is set to green which is the same color as the background color of the region R21, and the color of the bar meter B2 is set to red which is the same color as the background color of the region R22.

According to Modification 3, not only the background colors of the regions R11, R12 but also the colors of the bar meters B1, B2 are set to green and red, respectively. Thus, the subject can more clearly understand which of expiration and inspiration his/her respiratory motion is in. Therefore, the subject can more effectively conduct the swallowing training such that the subject performs swallowing at an ideal timing with respect to the respiration.

In a case where the colors of the bar meters B1, B2 indicate expiration and inspiration in this manner, the displays of the background colors of the regions R11, R12 may be omitted, or alternatively, the background colors of the regions R11 R12 may be the same color. However, as in Modification 3, when the background colors of the regions R11, R12 are set to be different between expiration and inspiration, together with the colors of the bar meter B1, the subject can more clearly understand his/her own respiratory motion.

### <Modification 4>

Each of FIG. 14(c) and FIG. 14(d) is a diagram showing a configuration of the screen D1 displayed on the display 111 according to Modification 4.

In Modification 4, through adjustment of the display of segment groups forming the bar meters B1, B2, the swallowing timing in the respiratory motion is displayed to the subject. More specifically, the segment farthest from the boundary between the regions R21 and R22, in the segment group displayed in the region R21, R22 at the timing when swallowing has been detected, is continued to be displayed for a certain period (for example, about 1 second) in a color that is different from the color of the other segments.

In FIG. 14(c), swallowing is detected at the timing when the bar meter of which rightmost segment is a segment B11 is displayed in the region R21. The state of the expiration at the current time point is indicated by the length and the moving direction of the bar meter B1 (excluding segment B11) displayed in the region R21 at the current time point. In FIG. 14(d), swallowing is detected at the timing when the bar meter of which leftmost segment is a segment B21 is displayed in the region R21. The state of the inspiration at the current time point is indicated by the length and the moving direction of the bar meter B2 (excluding segment B21) displayed in the region R22 at the current time point.

According to Modification 4, the timing of the swallowing just performed by the subject can be confirmed by the display of segment B11, B21. Thus, while the subject sequentially and clearly confirms the timing of swallowing with respect to respiration, the subject can conduct the swallowing training more effectively.

### <Modification 5>

In Modification 5, the color of the bar meter B1 displayed in the region R21 is changed between the first half and the second half of an expiration period. The color of the bar meter B1 is set to green in the first half of the expiration period, and the color of the bar meter B1 is set to yellow in the second half of the expiration period. With respect to an inspiration period, the color of the bar meter B2 is set to red in the entire period thereof.

In Modification 5, through the adjustment of the colors of the bar meters B1, B2 in this manner, a trigger for swallowing is provided to the subject. That is, the period in which the bar meter B1 is displayed in green (the first half of the expiration period) is the best period for swallowing. As a result of the bar meter B1 being displayed in green, the subject receives the trigger for swallowing, and is induced to perform swallowing. The period in which the bar meter B1 is displayed in yellow (the second half of the expiration period) is a period that has a risk of occurrence of inspiration after swallowing. The period in which the bar meter B2 is displayed in red (the inspiration period) is a period that is not appropriate for swallowing (a period having a high risk of aspiration).

In Modification 5, since swallowing is induced by the color of the bar meter B1, the inducing sound for inducing the subject to perform swallowing is not outputted.

In Modification 5, an average value of the expiration periods P1 (see FIG. 6(a)) obtained from five expirations preceding the expiration at the current time point is obtained as the expiration period for the expiration at the current time point. The period from when the start of the expiration has been detected until a period corresponding to a half of this average value elapses is set as the first half of the expiration period, and the period from the end time point of this first half until the end of the expiration is set as the second half of the expiration period.

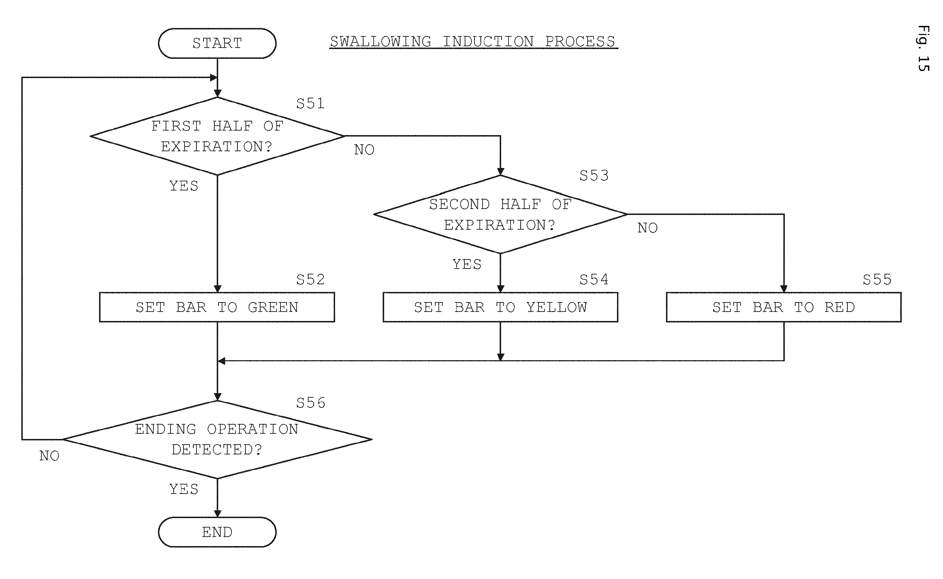

FIG. 15 is a flow chart showing a swallowing induction process according to Modification 5.

In S51, on the basis of the respiration data, the swallowing induction part 116c determines whether or not the respiratory motion at the current time point is included in the first half of the expiration period. When the determination in S51 is YES, the swallowing induction part 116c sets, in S52, the color of the bar meter B1 to green. When the determination in S51 is NO, the swallowing induction part 116c determines, in S53, whether or not the respiratory motion at the current time point is included in the second half of the expiration period. When the determination in S53 is YES, the swallowing induction part 116c sets, in S54, the color of the bar meter B1 to yellow. When the determination in S53 is NO, the swallowing induction part 116c sets, in S55, the color of the bar meter B2 to red. The swallowing induction part 116c repeats the processes of S51 to S55 until the swallowing induction part 116c determines, in S56, that the ending operation of the swallowing training has been inputted.

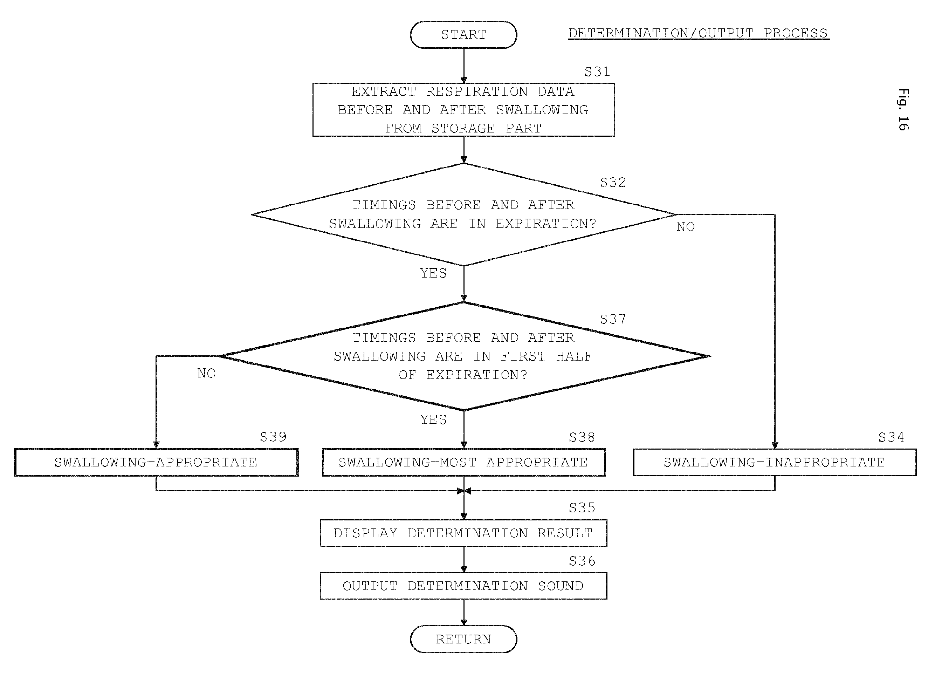

FIG. 16 is a flow chart showing a determination output process according to Modification 5. In the flow chart in FIG. 16, S33 in the flow chart in FIG. 4(a) is replaced by S37 to S39.

When the determination in S32 is YES (when both of the timing immediately before the swallowing and the timing immediately after the swallowing are included in the expiration period), the determination output part 116e further determines, in S37, whether or not both of the timing immediately before the swallowing and the timing immediately after the swallowing are included in the first half of the expiration period. When the determination in S37 is YES, the determination output part 116e obtains, in S38, a determination result that the swallowing was performed at the most ideal timing with respect to the respiration. When the determination in S37 is NO, the determination output part 116e obtains, in S39, a determination result that the swallowing was performed at an ideal timing with respect to the respiration. When the determination in S32 is NO, the determination output part 116e obtains, in S34, a determination result that the swallowing was not performed at an ideal timing with respect to the respiration, as in the flow chart shown in FIG. 4(a). In S35, the determination output part 116e performs displaying based on the determination result.

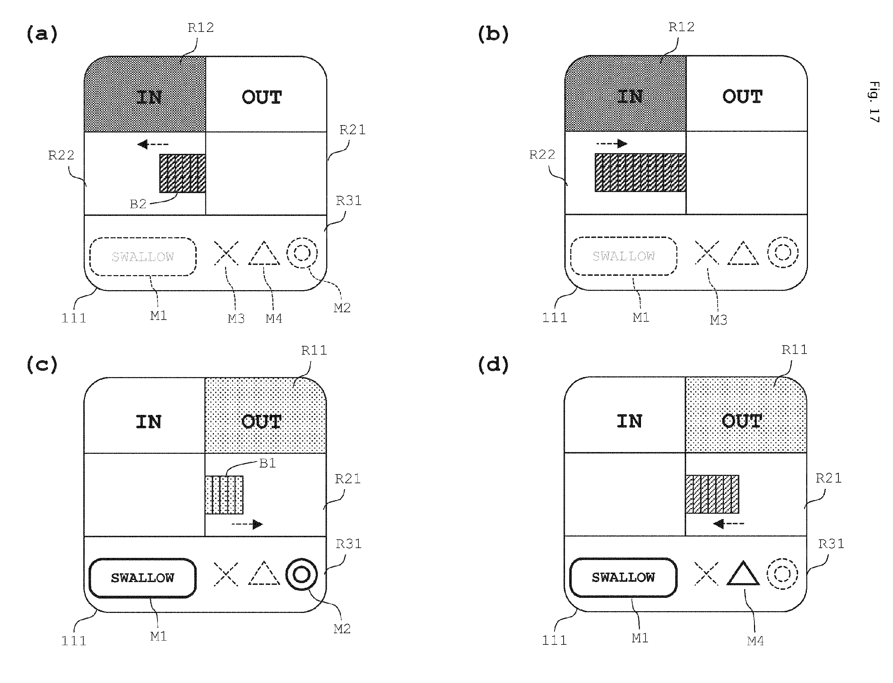

Each of FIG. 17(a) to FIG. 17(d) is a diagram showing a configuration of the screen D1 displayed on the display 111 according to Modification 5. In Modification 5, the region R31 includes a mark M4 indicating that the swallowing is not the most ideal but appropriate, in addition to the mark M2 indicating that the swallowing is the most ideal. The mark M3 is a mark indicating that the swallowing is not appropriate.

As shown in FIG. 17(a) and FIG. 17(b), when the respiratory motion is in an inspiration period, the bar meter B2 is displayed in red, in the region R22, which is the same color as the background color of the region R12. It should be noted that, when the subject performed swallowing in an inspiration period, the mark M1 and the mark M3 are displayed in the region R31.

As shown in FIG. 17(c), when the respiratory motion is in the first half of an expiration period, the bar meter B1 is displayed in green, in the region R21, which is the same color as the background color of the region R11. When the subject performs swallowing at this timing, the mark M1 and the mark M2 are displayed in the region R31 as shown in FIG. 17(c).

As shown in FIG. 17(d), when the respiratory motion is in the second half of the expiration period, the bar meter B1 is displayed in yellow, in the region R21, which is a color different from the background color of the region R11. When the subject performs swallowing at this timing, the mark M1 and the mark M4 are displayed in the region R31 as shown in FIG. 17(d).

According to Modification 5, in the period of the first half, of the expiration period, in which the most ideal swallowing timing is realized, the color of the bar meter B1 is set to green; and in the remainder of the expiration period, the color of the bar meter B1 is set to yellow. Accordingly, the subject can understand his/her own respiratory motion on the basis of the bar meter B1, B2 (the second motion information), and can simultaneously receive a trigger for swallowing from the color of the bar meter B1. Further, on the basis of the mark M2, M4, the subject can understand whether or not his/her own swallowing was performed at the most ideal timing.

### <Modification 6>

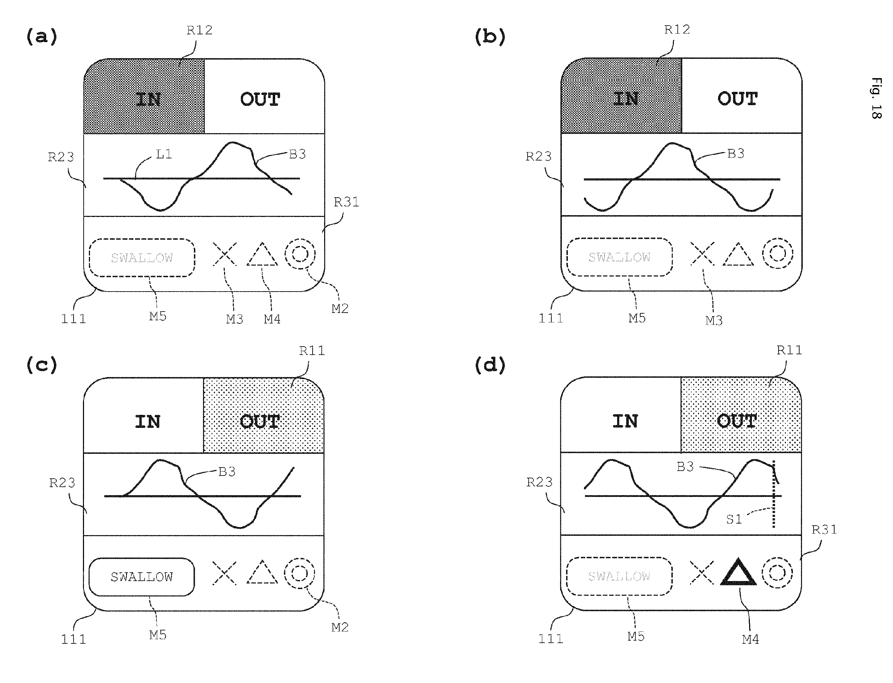

Each of FIG. 18(a) to FIG. 18(d) is a diagram showing a configuration of the screen D1 displayed on the display 111 according to Modification 6.

In Modification 6, the regions R21, R22 are replaced by a region R23. On the basis of the respiration data, the respiration output part 116b causes an image in which a respiration waveform B3 of the subject is superposed on a straight line L1 separating expiration and inspiration from each other, to be displayed in the region R23. The respiration waveform B3 shifts in association with the respiratory motion of the subject. In addition, the swallowing detection part 116d causes a broken-line mark S1, which indicates the timing of the detection of the swallowing, to be displayed so as to be superposed on the respiration waveform B3. The mark S1 also shifts together with the respiration waveform B3, in association with the respiratory motion of the subject.

Further, in the region R31, a mark M5 for inducing swallowing is displayed instead of the mark M1 which indicates occurrence of swallowing. At the swallowing induction timing, the swallowing induction part 116c causes the mark M5 to be displayed in the region R31. Here, the mark M5 is continued to be displayed in the entire period from when the expiration has been started until the first half of the expiration period ends. The marks M2 to M4 are displayed through processes similar to those in the cases shown in FIG. 17(a) to FIG. 17(d).

As shown in FIG. 17(c), the mark M5 is displayed in accordance with the start of expiration. Accordingly, swallowing is induced in the subject. As shown in FIG. 18(d), when the first half of the expiration period ends, the mark M5 is no longer displayed. When the subject performs swallowing in the second half of the expiration period, the mark S1 indicating the swallowing timing is displayed so as to be superposed on the respiration waveform B3, and further, the mark M4 is displayed in the region R31, as shown in FIG. 18(d).

According to Modification 6, since the respiration waveform B3 is displayed, the subject can understand in a simple manner the state of his/her own respiration, together with the shift of the respiration, and can easily perform swallowing so as to meet the timing of the swallowing induction trigger (display of the mark M5). Therefore, the subject can smoothly conduct the swallowing training.

### <Other modifications>

Methods for detecting respiration have been described in the embodiment and Modifications 1, 2. However, respiration may be detected by still another method. For example, respiration may be detected on the basis of a respiration sound of the subject.

The swallowing inducing sound may be a monotonous sound such as a buzzer sound, or alternatively, words that prompt swallowing may be outputted in terms of voice. The first sound, which notifies that the swallowing is appropriate, is preferably a sound that allows intuitive recognition of the appropriateness, and the second sound, which notifies that the swallowing is not appropriate, is preferably a sound that allows intuitive recognition of the inappropriateness. Also, the first sound and the second sound may respectively be voices that notify appropriateness and inappropriateness of the swallowing, in terms of words.

The contents and the layout of the screen D1 are not limited to those described above. For example, instead of the bar meters B1, B2, the state of respiration may be displayed in terms of rotation of a pointer. In addition, expiration and inspiration may be announced in terms of sounds similar to an expiration sound and an inspiration sound, respectively, or in terms of other sounds.

In addition to the above, various modification can be made as appropriate to the embodiment of the present invention, without departing from the scope of the technical idea defined by the claims.

### DESCRIPTION OF THE REFERENCE CHARACTERS

100 swallowing training apparatus
111 display
113 sound output part
114 pressure sensor (respiration detection part)
116 controller
116a respiration detection part
116b respiration output part
116c swallowing induction part
116d swallowing detection part
116e determination output part (swallowing determination part, result output part)
120 nasal cannula (respiration detection part)
131a sound sensor (sound detection part)
131b displacement sensor (displacement detection part)
140 detection band (respiration detection part)
140a strain sensor
150 respiration detector (respiration detection part)
151c, 151d temperature sensor (respiration detection part)
B1, B2 bar meter (indicator)

## Claims

1. A swallowing training apparatus for swallowing training, the swallowing training apparatus comprising:
a respiration detection part configured to detect a respiratory motion of a subject;
a swallowing induction part configured to provide a trigger for swallowing induction to the subject on the basis of a detection result from the respiration detection part;
a swallowing detection part configured to detect that swallowing has been performed by the subject;
a swallowing determination part configured to determine whether or not the swallowing detected by the swallowing detection part has been at an appropriate timing with respect to the respiratory motion detected by the respiration detection part; and
a result output part configured to output, to outside, information indicating a determination result, in accordance with determination having been performed by the swallowing determination part.

2. The swallowing training apparatus according to claim 1, further comprising
a respiration output part configured to output, to outside, information indicating the respiratory motion of the subject on the basis of the detection result from the respiration detection part.

3. The swallowing training apparatus according to claim 2, further comprising
a display configured to display information, wherein
the respiration output part causes the display to display, as the information indicating the respiratory motion: first motion information indicating which of expiration and inspiration the respiratory motion is in; and second motion information indicating a progress state of expiration and inspiration.

4. The swallowing training apparatus according to claim 3, wherein
the respiration output part causes display of at least one of the first motion information and the second motion information to be different between a case of the expiration and a case of the inspiration.

5. The swallowing training apparatus according to claim 4, wherein
the respiration output part causes a color of the display of at least one of the first motion information and the second motion information to be different between the case of the expiration and the case of the inspiration.

6. The swallowing training apparatus according to claim 5, wherein
the respiration output part sets the color of the display in the case of the expiration to green, and sets the color of the display in the case of the inspiration to red.

7. The swallowing training apparatus according to any one of claims 3 to 6, wherein
as the second motion information, the respiration output part causes the display to display strength of the expiration by means of an indicator configured to change in a first direction, and causes the display to display strength of the inspiration by means of an indicator configured to change in a second direction opposite to the first direction.

8. The swallowing training apparatus according to any one of claims 3 to 6, wherein
as the second motion information, the respiration output part causes the display to display an image in which a respiration waveform is superposed on a straight line separating the expiration and the inspiration from each other.

9. The swallowing training apparatus according to any one of claims 3 to 8, wherein
the result output part causes the display to display a determination result on appropriateness or inappropriateness of the swallowing, together with the first motion information and the second motion information.

10. The swallowing training apparatus according to claim 9, further comprising
a sound output part configured to output a sound, wherein
the result output part causes the sound output part to output a sound indicating the determination result on appropriateness or inappropriateness of the swallowing.

11. The swallowing training apparatus according to any one of claims 3 to 10, further comprising
a swallowing output part configured to cause the display to display information indicating occurrence of swallowing, in accordance with the swallowing detection part having detected the swallowing.

12. The swallowing training apparatus according to any one of claims 1 to 11, wherein
the swallowing induction part provides the trigger to the subject at a predetermined timing in a first half of an expiration period.

13. The swallowing training apparatus according to any one of claims 3 to 11, wherein
the swallowing induction part provides the trigger to the subject by causing a display form of the second motion information in an ideal swallowing period in an expiration period to be different from a display form of a remainder of a respiration period.

14. The swallowing training apparatus according to any one of claims 1 to 13, comprising
a sound detection part configured to detect a sound of a larynx portion of the subject, wherein
the swallowing detection part detects the swallowing on the basis of respiration information detected by the respiration detection part and sound information detected by the sound detection part.

15. The swallowing training apparatus according to claim 14, further comprising
a displacement detection part configured to detect a displacement of the larynx portion of the subject, wherein
the swallowing detection part detects the swallowing on the basis of the respiration information detected by the respiration detection part, the sound information detected by the sound detection part, and displacement information detected by the displacement detection part.
